# EUROPEAN PATENT APPLICATION

(11) **EP 4 706 751 A1**
(43) Date of publication of application: **11.03.2026**
(21) Application number: 25200676.2
(22) Date of filing: 05.09.2025
(51) Int. Cl.: A61N 1/375, H01R 13/187, H01R 24/58

(54) **A HERMETIC LEAD CONNECTOR MADE BY DIFFUSION BONDING AN INTERMEDIATE CERAMIC RING TO OPPOSED CONDUCTIVE CONTACT RINGS**

(30) Priority: 06.09.2024 US 202463691597 P; 15.11.2024 US 202463720816 P; 02.09.2025 US 202519316471
(71) Applicant: Greatbatch Ltd., Clarence, NY 14031 (US)
(72) Inventor: RUBINO, Robert, Williamsville, 14221 (US); GANGULY, Adrish, Clarence, 14031 (US); KOCH, Melissa, Spencerport, 14559 (US); ZHOU, Boyang, Buffalo, 14215 (US); VILLAMIL, Daniel, CP11400 Montevideo (UY); DUARTE, Camila, Montevideo (UY)
(74) Representative: Mathys & Squire

(57) **Abstract**

A modular lead connector for a medical device is described. The modular lead connector comprises an alumina ring having opposed first and second faces provided with respective first and second metallizations. First and second gold washers are contacted to the respective first and second metallizations, and the ceramic ring supporting the first and second gold washers is positioned between first and second titanium contact rings. Then, with the ceramic ring and the titanium contact rings being in axial alignment, they are hermetically connected to each other at respective first and second titanium/gold/alumina diffusion bonds. A lead connector assembly comprising at least two modular lead connectors in axial alignment and welded together is also described. An open sleeve is connected to a proximal-most one of the axially aligned modular lead connectors, and a terminal plate is welded to a distal-most one of the axially aligned modular lead connectors.

## Description

### CROSS-REFERENCE TO RELATED APPLICATIONS

This application claims priority to U.S. Provisional Application Serial Nos. 63/691,597, filed on September 6, 2024, and 63/720,816, filed on November 15, 2024, as well as U.S. Patent Application Serial No. 19/316,471 filed on September 2, 2025.

### FIELD OF THE INVENTION

The present invention generally relates to the field of diffusion bonding of dissimilar materials. More particularly, the present invention relates to implantable medical devices, such as pacemakers, defibrillators, and nerve stimulators that require both a hermetic seal to prevent the intrusion of biological fluids and other foreign materials into the device and that have at least one hermetically sealed conductive pathway which allows an electronic signal to pass from inside to the outside of the medical device.

These objectives are achieved using a connector module that is made by diffusion bonding an electrically non-conductive ceramic ring supporting opposed gold washers between opposed electrically conductive rings. Alumina (Al₂O₃) and sapphire are suitable electrically non-conductive materials for diffusion bonding to a conductive material such as a titanium or a titanium alloy ring. At least two of the connector modules may be aligned end-to-end and connected to each other at abutting electrically conductive rings to form a hermetic lead connector for the medical device. An electrically conductive canted coil spring may be seated in a recess between adjacent connector modules. An implantable lead can then be inserted into the lead connector to connect the lead to the medical device.

### BACKGROUND OF THE INVENTION

Implantable medical devices, such as pacemakers, defibrillators, and nerve stimulators require both a hermetic seal to prevent the intrusion of biological fluids and other foreign materials into the device and at least one hermetically sealed conductive pathway which allows electrical signals to pass between the device electronics and body tissue. These objectives are typically achieved by connecting an implantable lead to a medical device that is configured to send and receive electrical pulses or signals to and from body tissue. The implantable lead has a proximal end comprising at least two spaced-apart electrical contacts. The electrical contacts are connected to distal electrodes of the implantable lead that are configured to electrically stimulate body tissue and to send electrical signals related to the functioning of body tissue back to the lead's proximal electrical contacts.

In a functioning system, the proximal electrical contacts of the implantable lead are connected to a lead connector portion of the medical device. Terminal blocks in the lead connector connect to the lead's proximal electrical contacts. The terminal blocks in turn are connected to the terminal pins of a hermetic feedthrough supported by the medical device. The feedthrough terminal pins serve as electrically conductive pathways into the interior of the medical device where the pins are connected to internal electronic circuitry. Typically, the internal circuitry is programmed to generate electrical stimulation pulses that travel distally through the terminal pins to the terminal blocks of the lead connector and then to the lead electrodes for electrical stimulation of body tissue and to receive electrical signals back from the body tissue.

The feedthrough terminal pins and lead connector as an assembly are typically housed inside a molded header made from an insulative polymeric material supported on the medical device. Molded headers are commonly made from medical grade thermoplastic materials such as adhesives, epoxies, and other such sealants. A thermoplastic polyurethane distributed by Lubrizol Advanced Materials, Inc. under the trademark TECOTHANE^{®} is a commonly used material for manufacturing medical device headers.

However, as the drive to make medical devices smaller and smaller without compromising functionality advances, there is a strong desire to eliminate the molded header. Not only does the header add bulk to the medical device, but it also significantly decreases production yields as curing processes for its medical grade thermoplastic materials are prone to defects.

### SUMMARY OF THE INVENTION

Aspects and embodiments of the present invention are set out in the appended claims.

The purpose of the present invention is to simplify the electrical connections between the internal electronic circuitry housed inside a medical device and the electrical contacts at the proximal end of an implantable lead. This is done by forming a lead connector into which electrical contacts at the proximal end of an implantable lead connect. The electrical contacts of the lead connector are formed by diffusion bonding a biocompatible material, such as an FDA approved biocompatible material such as alumina (Al₂O₃) or sapphire or other ceramic, supporting a gold washer on its major face to a conductive material such as titanium or a titanium alloy. Titanium forms part of a terminal block that electrically connects between an electrical contact at the proximal end of an implantable lead and a jumper wire that is connected to internal device circuitry. Alumina or sapphire electrically isolate immediately adjacent titanium terminal blocks from each other. However, the jumper wire is not part of a hermetic feedthrough as is well known by those skilled in the art.

In that respect, the present invention relates to a modular lead connector for a medical device. The modular lead connector comprises first and second electrically conductive contact rings, a ceramic ring having opposed first and second faces provided with respective first and second metallizations, and first and second gold washers contacted to the respective first and second metallizations. The ceramic ring supporting the first and second gold washers is intermediate the first and second electrically conductive contact rings. The ceramic ring is in axial alignment with and hermetically connected to the respective first and second electrically conductive contact rings at respective first and second diffusion bonds.

The first and second metallizations may each have a thickness that ranges from about 10 nanometers to less than 1µ.

The first and second gold washers may each have a thickness of about 50µ.

The ceramic ring may comprise an inner annular surface spaced from an outer annular surface. The inner and outer annular surfaces may be concentric and extending to opposed first and second planar faces provided with the respective first and second metallizations.

The first and second electrically conductive contact rings may comprise titanium or titanium alloys and the ceramic ring may comprise alumina or sapphire.

The first and second hermetic diffusion bonds of the ceramic ring supporting the first and second gold washers intermediate the respective first and second electrically conductive contact rings may each have a leak rate that ranges from about 0.2 to about 3.4 x 10⁻¹⁰ std cc He/s.

The ceramic ring intermediate the first and second electrically conductive contact rings may each have the same outer diameter that ranges from about 1,000µ to about 5,000µ. An inner diameter of the intermediate ceramic ring may be from about 2% to about 15% larger than the inner diameters of the first and second electrically conductive contact rings, which may be the same and range from about 500µ to about 2,000µ.

An O-ring may be seated against an inner annular surface at the inner diameter of the ceramic ring and confined in position by the first and second electrically conductive contact rings.

The first and second electrically conductive contact rings may have respective first and second planar inner surfaces facing the intermediate ceramic ring. Respective first and second raised annular alignment protrusions may face each other and extend in front of the inner annular surface of the intermediate ceramic ring.

The present invention further relates to a lead connector assembly for a medical device. The lead connector assembly comprises at least two modular lead connectors, each modular lead connector comprises first and second titanium contact rings, an alumina ring having opposed first and second faces provided with respective first and second metallizations, and first and second gold washers contacted to the respective first and second metallizations. The alumina ring supporting the first and second gold washers is intermediate the first and second titanium contact rings. The alumina ring is in axial alignment with and hermetically connected to the respective first and second titanium contact rings at respective first and second diffusion bonds. At least two modular lead connectors are axially aligned and welded together. An open sleeve is connected to a proximal-most one of the at least two axially aligned modular lead connectors, and a terminal plate is welded to a distal-most one of the at least two axially aligned modular lead connectors.

The first and second metallizations provided on the opposed first and second faces of the alumina ring for the at least two axially aligned modular lead connectors may each have a thickness that ranges from about 10 nanometers to less than 1µ.

The first and second gold washers contacted to the respective first and second metallizations provided on the opposed first and second faces of the alumina ring for each of the at least two axially aligned modular lead connectors may have a thickness of about 50µ.

The alumina ring for each of the at least two axially aligned modular lead connectors may comprise an inner annular surface spaced from an outer annular surface. The inner and outer annular surfaces may be concentric and extend to opposed first and second planar faces provided with the respective first and second metallizations.

The first and second hermetic diffusion bonds of the alumina ring supporting the first and second gold washers intermediate the respective first and second titanium contact rings for the at least two axially aligned modular lead connectors may each have a leak rate that ranges from about 0.2 to about 3.4 x 10⁻¹⁰ std cc He/s.

The first and second titanium contact rings and the intermediate alumina ring may each have the same outer diameter that ranges from about 1,000µ to about 5,000µ. An inner diameter of the intermediate alumina ring may be from about 2% to about 15% larger than the inner diameters of the first and second titanium contact rings, which may be the same and range from about 500µ to about 2,000µ.

An O-ring may be seated against an inner annular surface at the inner diameter of the alumina ring and confined in position by the first and second titanium contact rings.

The first and second titanium contact rings may have respective first and second planar inner surfaces facing the intermediate alumina ring. Respective first and second raised inner annular alignment protrusions may face each other and extend radially inwardly in front of the inner annular surface of the intermediate alumina ring.

The at least two axially aligned modular lead connectors welded together may comprise a first modular lead connector and an adjacent second modular lead connector. The first titanium contact ring of the first modular lead connector may have a first raised outer annular alignment protrusion that is received in a second outer annular step of the second titanium contact ring of the adjacent second modular lead connector. The second titanium contact ring of the second modular lead connector may further comprise a second intermediate inner annular step that is spaced radially inwardly from the second outer annular step and which meets a second beveled annular surface which in turn meets a second inner annular step that is spaced radially inwardly from the second beveled annular surface. An annular metallic contact spring may be seated against the second beveled annular surface extending to the opposed second intermediate inner annular step and the second inner annular step of the second modular lead connector and the first raised outer annular alignment protrusion of the first titanium contact ring of the fist modular lead connector.

Still further, the present invention relates to an active medical device (AMD). The AMD comprises a device housing having a housing sidewall defining an open housing interior. The housing sidewall extends to a device housing annular rim. A printed circuit board (PCB) assembly comprising a printed circuit board supporting at least one electronic component is housed inside the housing interior. An electrical power source housed inside the housing interior is connected to the PCB assembly. A housing cap portion of the device housing comprises a housing cap sidewall defining an open housing cap interior. The housing cap sidewall extends to a housing cap annular rim. The housing cap sidewall comprising a lead opening.

The AMD also has a lead connector assembly that comprises at least two modular lead connectors. Each modular lead connector comprises first and second titanium contact rings, an alumina ring having opposed first and second faces provided with respective first and second metallizations, and first and second gold washers contacted to the respective first and second metallizations. The alumina ring supporting the first and second gold washers is intermediate the first and second titanium contact rings. The alumina ring is in axial alignment with and hermetically connected to the respective first and second titanium contact rings at respective first and second diffusion bonds, and the at least two modular lead connectors are axially aligned and welded together. An open sleeve is connected to a proximal-most one of the at least two axially aligned modular lead connectors, and a terminal plate is welded to a distal-most one of the at least two axially aligned modular lead connectors. That way, the open sleeve of the lead connector assembly is welded to an interior surface of the housing cap aligned with the lead opening. At least one jumper wire extends from at least one of a second titanium contact ring welded to a first titanium contact ring of the respective at least two modular lead connectors to the PCB assembly housed inside the housing interior. The PCB assembly is also connected to the electrical power source.

Further, a communication antenna/inductive charging coil assembly may be electrically connected to the at least one electronic component of the PCB assembly.

These and other objectives of the present invention will become increasingly more apparent to those skilled in the art after having read the following detailed description in light of the appended drawings.

### BRIEF DESCRIPTION OF THE DRAWINGS

FIG. 1 is an exploded view of a lead connector 10 according to the present invention.
FIG. 1A is a perspective view of the lead connector assembly 10 shown in FIG. 1.
FIG. 2 is a cross-sectional view taken along line 2-2 of the lead connector assembly 10 shown in FIGS. 1 and 1A.
FIG. 3 is a perspective view of a first titanium contact ring 14 for the lead connector assembly 10 shown in FIGS. 1, 1A and 2.
FIG. 3A is a cross-sectional view taken along line 3A-3A of the first titanium contact ring 14 shown in FIG. 3.
FIG. 4 is a perspective view of a second titanium contact ring 16 for the lead connector assembly 10 shown in FIGS. 1, 1A and 2.
FIG. 4A is a cross-sectional view taken along line 4A-4A of the second titanium contact ring 16 shown in FIG. 4.
FIG. 4B is an enlarged view of FIG. 4A showing the angular relationships of the indicated surfaces for the contact ring 16.
FIG. 5 is a perspective view of an intermediate ceramic ring 18 for the lead connector assembly 10 shown in FIGS. 1, 1A and 2.
FIG. 5A is a cross-sectional view taken along line 5A-5A of the intermediate ceramic ring 18 shown in FIG. 5.
FIG. 6 is a perspective view of the intermediate ceramic ring 18 shown in FIG. 5 with gold washers 20A, 20B contacted to its opposed major faces 18A, 18B.
FIG. 6A is a cross-sectional view taken along line 8A-8A of the intermediate ceramic ring 18 with the gold washers 20A, 20B shown in FIG. 6.
FIG. 7 is a perspective view of an exemplary fixture 300 for diffusion bonding a first titanium contact ring 14/an intermediate ceramic ring 18 supporting gold washers 20A, 20B/a second titanium contact ring 16 subassembly to form the modular lead connector 12 (FIG. 1) according to the present invention.
FIG. 8 a perspective view of another exemplary fixture 400 for diffusion bonding a first titanium contact ring 14/an intermediate ceramic ring 18 supporting gold washers 20A, 20B/a second titanium contact ring 16 subassembly to form a modular lead connector 12 (FIG. 1) according to the present invention.
FIG. 9 shows a subassembly comprising the first titanium contact ring 14/the intermediate ceramic ring 18 supporting gold washers 20A, 20B/the second titanium contact ring 16 before the rings are contacted to each other in one of the fixtures 300, 400 depicted in FIGS. 7 and 8.
FIG. 9A shows a modular lead connector 12 which results from diffusion bonding the first titanium contact ring 14/the intermediate ceramic ring 18 supporting gold washers 20A, 20B/the second titanium contact ring 16 subassembly shown in FIG. 9.
FIG. 10 is a cross-sectional view along line 10-10 of FIG.9A. This drawing shows an enlarged view of the modular lead connector 12 including an O-ring 22 seated in the space between the annular protrusion 14E of the first contact ring 14 and the inner annular surface 16J of the second contact ring 16.
FIG. 11 is a scanning electron microscope (SEM) image showing the interface of a titanium contact ring 14, 16, an intermediary gold washer 20A, 20B, and an alumina ring 18.
FIG. 12 is an SEM image showing the magnified interface between a titanium contact ring 14, 16 and a gold washer 20A, 20B with undisturbed titanium and undisturbed gold on opposed sides of the diffusion bond layer between the two bulk materials according to the present invention.
FIG. 13 is an SEM image showing the magnified diffusion bond interface between an alumina ring 18 and a gold washer 20A, 20B with undisturbed alumina and undisturbed gold on opposed sides of the diffusion bond layer between the two bulk materials according to the present invention.
FIG. 14 is a broken-apart view of an exemplary active medical device (AMD) 30 according to the present invention.
FIG. 14A is a side view of a lead connector assembly 10 connected to a printed circuit board (PCB) 36 for the AMD shown in FIG. 14 according to the present invention.
FIG. 15 is an enlarged elevational view of a lead connector assembly 10 connected to a housing cap 34 for the AMD 30 shown in FIGs. 14 and 14A.
FIG. 16 is an end elevational view of the lead connector assembly 10 shown in FIG. 15.
FIG. 17 is a schematic view of an exemplary embodiment of a lead connector assembly 10 including an open sleeve 44 for connecting the lead connector assembly to the housing cap 34 shown in FIGS. 14, 14A, 15 and 16.
FIG. 18 is a broken-apart view of an exemplary active medical device (AMD) 30A including a PCB assembly 36 supporting a communication antenna/inductive charging coil assembly 58 according to the present invention.
FIGS. 19 and 20 show alternate embodiments for exemplary main housings 32B and 32C for an AMD according to the present invention.

### DETAILED DESCRIPTION OF THE PREFERRED EMBODIMENTS

In this specification, the term "diffusion bond" is defined as a solid-state monolithic atomic bond at the interfaces of ceramic and titanium members in contact with gold washers due to local plastic deformation at elevated pressure and temperature. Elevated pressure and temperature aid interdiffusion at the interfacial surface contact of the ceramic and titanium materials being joined. In the present invention, a diffusion bond is created between bulk materials comprising a first titanium contact ring 14, a spaced-apart second titanium contact ring 16, and an intermediate ceramic ring 18 faced with intermediary gold washers by diffusion of titanium from the contact rings 14, 16 into the intermediary gold washers, and by diffusion of ceramic 18 from its opposed major faces into both intermediary gold washers.

Further, the term "diffusion bond thickness" is measured from either: (i) a plane aligned along the contact interface between the ceramic ring and the intermediary gold washers, and (ii) a plane aligned along the contact interface between the opposed titanium contact rings and the intermediary gold washers, and includes the combined depth of the bond extending outwardly from that plane along an x-axis into undisturbed portions of both (i) the ceramic and gold washers, and (ii) the opposed titanium contact rings and gold washers.

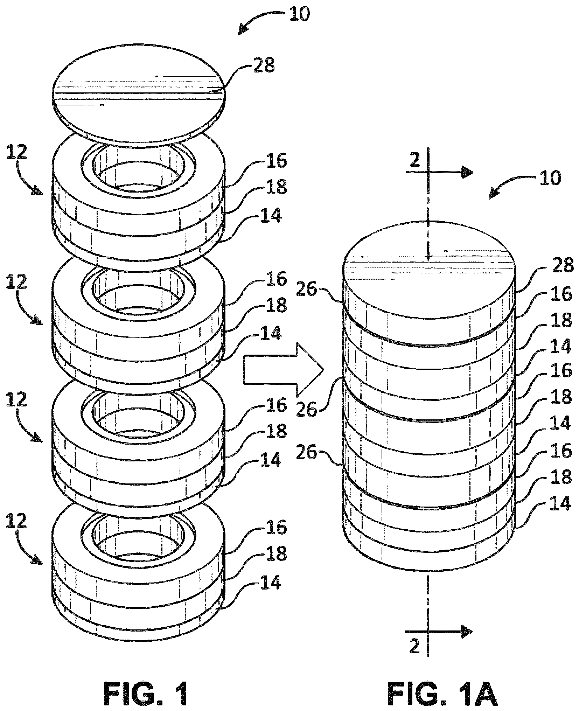

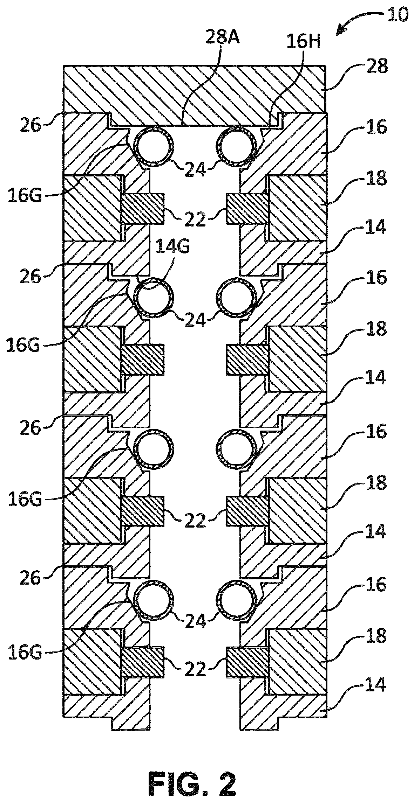

Turning now to the drawings, FIGS. 1, 1A and 2 illustrate a lead connector assembly 10 according to the present invention. The lead connector assembly 10 is constructed from a series of modular lead connectors 12 that are fitted together and then hermetically sealed to each other as an assembly. The lead connector assembly 10 can comprises from as few as two modular lead connectors 12 to many of them, for example, 20 or more modular connectors 12.

Each modular lead connector 12 is comprised of a first contact ring 14 made from an electrically conductive metal, a spaced-apart second contact ring 16 made from an electrically conductive metal, and an intermediate ring 18 made from an insulating ceramic. The ceramic ring 18 resides between and is connected to the first and second electrically conductive contact rings 14, 16. It is noted that alumina is an exemplary insulating ceramic, but other ceramic materials such as sapphire are suitable for practicing the present invention. Titanium or an alloy of titanium is a suitable contact metal for the first and second contact rings 14, 16.

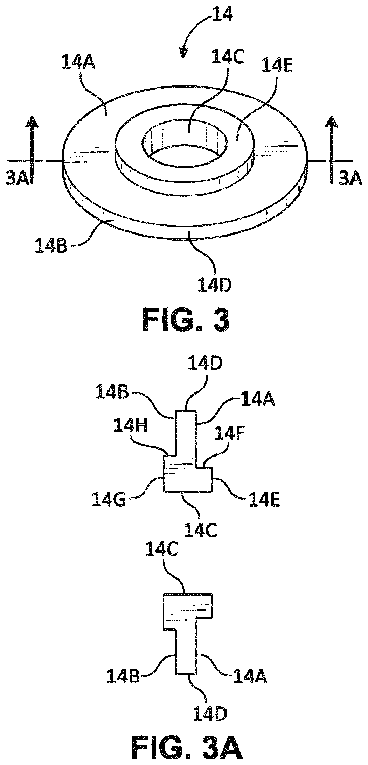

As shown in FIGS. 3 and 3A, the first titanium contact ring 14 has a planar upper face 14A that is spaced from and parallel to a planar lower face 14B. The upper and lower faces 14A, 14B extend to an inner annular edge 14C and an opposed outer annular edge 14D. The inner and outer annular edges 14C, 14D are preferably circular and concentric.

The distance from the planar upper face 14A to the planar lower face 14B ranges from about 500µ to about 1,000µ and defines the thickness of the first contact ring 14. Further, the distance from the inner edge 14C to the outer edge 14D defines its width. The inner diameter of the inner annular edge 14C ranges from about 500µ to about 2,000µ, and the outer diameter of the outer annular edge 14D ranges from about 1,000µ to about 5,000µ, This means that the width of the first titanium contact ring 14 ranges from about 500µ to about 3,000µ (calculated as the difference between the inner diameter ranging from about 500µ to about 2,000µ and the outer diameter ranging from about 1,000µ to about 5,000µ).

The planar upper face 14A of the first contact ring 14 is interrupted by a raised upper annular alignment protrusion 14E meeting the inner annular edge 14C. An upper annular step 14F resides where the upper alignment protrusion 14E meets the upper face 14A. Similarly, the planar lower face 14B is interrupted by a raised lower annular alignment protrusion 14G meeting the inner annular edge 14C. A lower annular step 14H resides where the lower protrusion 14G meets the lower face 14B. The annular protrusions 14E and 14G are about 60% as wide as the width of the first titanium contact ring 14, and each alignment protrusion 14E, 14G has a thickness measured from its respective planar face 14A, 14B to an outer planar surface of the protrusion 14E, 14G. The outer planar surfaces of the protrusions 14E, 14G are parallel to the respective planar face 14A, 14B from which they extend. The alignment protrusions 14E, 14G are about 40% to about 80% as thick as the contact ring 14. The upper and lower alignment protrusions 14E and 14G can have similar or different annular shapes. Different annular shapes for the protrusions 14E and 14G are shown in FIG. 3A.

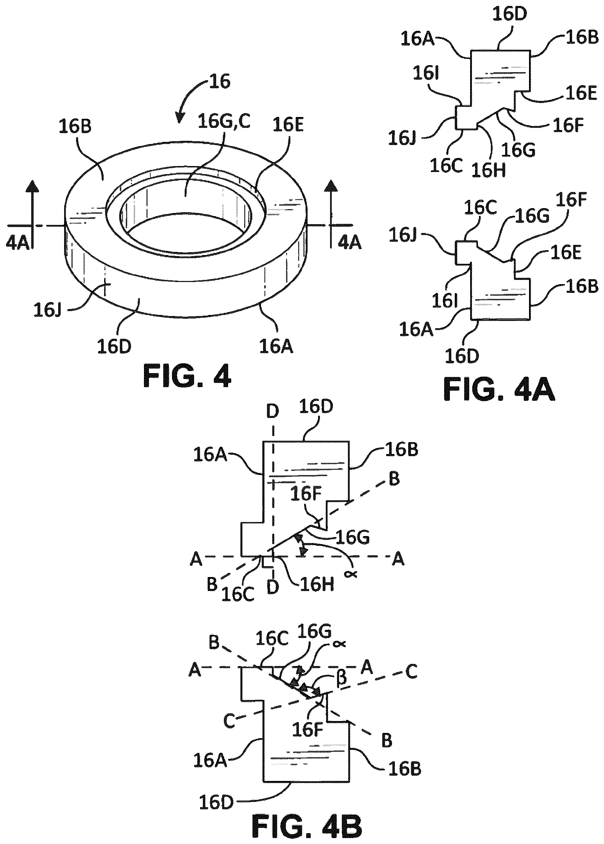

As shown in FIGS. 4 and 4A, the second titanium contact ring 16 has a planar lower face 16A spaced from and parallel to a planar upper face 16B. The lower and upper faces 16A, 16B extend to an inner annular edge 16C. A circular outer annular edge 16D is concentric with the inner annular edge 16C.

The distance from the lower face 16A to the upper face 16B ranges from about 500µ to about 1,000µ and defines the thickness of the second titanium contact ring 16. Further, the distance from the inner edge 16C to the outer edge 16D defines its width. The inner diameter of the inner annular edge 16C ranges from about 500µ to about 2,000µ, and the outer diameter of the outer annular edge 16D ranges from about 1,000µ to about 5,000µ, This means that the width of the second titanium contact ring 16 ranges from about 500µ to about 3,000µ (calculated as the difference between the inner diameter ranging from about 500µ to about 2,000µ and the outer diameter ranging from about 1,000µ to about 5,000µ). Regardless, the thickness and width of the second contact ring 16 is the same as that of the first contact ring 14.

The planar upper face 16B of the second titanium contact ring 16 meets a first inner annular step 16E which extends inwardly to a second inner annular step 16F that meets a beveled annular surface 16G which in turn extends downwardly and inwardly to meet a third inner annular step 16H at the inner annular edge 16C. The significance of the contoured shape comprising the second annular step 16F, the beveled annular surface 16G and the third annular step 16H for the second titanium contact ring 16 will be described in detail hereinafter. Further, the lower face 16A extends inwardly to a fourth annular step 16I which in turn meets an inner annular protrusion having an inner annular surface 16J. The inner annular surface 16J of the inner protrusion is parallel to and concentric with the planar upper face 16B.

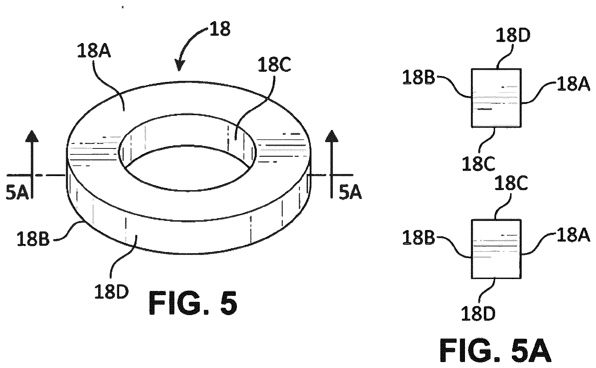

As shown in FIGS. 5 and 5A, the intermediate ceramic ring 18 has a planar upper or first face 18A spaced from and parallel to a planar lower or second face 18B. The upper and lower faces 18A, 18B extend to an inner annular edge 18C and an outer annular edge 18D. The inner and outer annular edges 18C, 18D are preferably circular and concentric. The distance from the upper face 18A to the lower face 18B defines the thickness of the ceramic ring 18, which ranges from about 300µ to about 2,000µ, while the distance from the inner edge 18C to the outer edge 18D defines its width.

The outer diameter of the ceramic ring 18 matches that of the first and second titanium contact rings 14, 16 and ranges from about 1,000µ to about 5,000µ. The inner diameter of the ceramic ring 18 is from about 2% to about 15% larger than the inner diameters of the inner annular edges 14C, 16C of the titanium contact rings 14 and 16.

Prior to positioning the ceramic ring 18 between the first and second titanium contact rings 14 and 16, its planar upper and lower faces 18A, 18B are provided with a metallization (not shown). A suitable metallization comprises two metallization layers including a first adhesion layer that is directly applied to the planar faces 18A, 18B, and a second, wetting layer, which is applied on top of the adhesion layer. In a preferred embodiment, the adhesion layer is titanium, and the wetting layer is either niobium or molybdenum with niobium being preferred. The metallization layers supported on the planar upper and lower faces 18A, 18B of the ceramic ring each have a thickness ranging from about 10 nanometers to less than 1µ.

The adhesion and wetting metallization layers may be applied to the planar faces 18A, 18B of the ceramic ring 18 by thin and thick film technologies, such as printing, painting, plating, and deposition processes. Metallization processes include screen printing, pad printing, brush coating, direct bonding, active metal brazing, magnetron sputtering, physical vapor deposition, ion implantation, electroplating, and electroless plating. In an alternate embodiment, both the adhesion and wetting metallization layers may be provided by a single metallization layer. Importantly, care must be taken to not contact any metallization to the inner and outer annular edges 18C, 18D of the ceramic ring 18. It is noted that in the present drawings, the adhesion and wetting layers are intentionally not shown for the sake of simplicity.

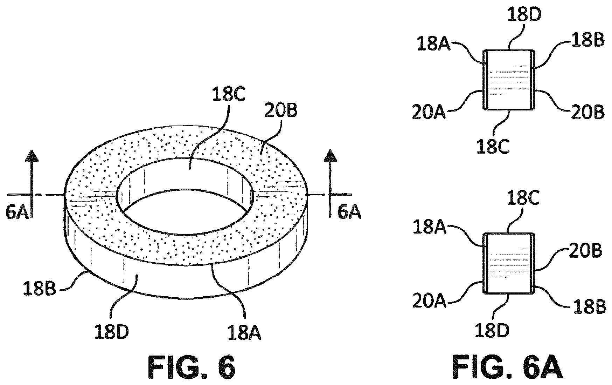

As shown in FIGS. 6 and 6A, after the planar upper and lower faces 18A, 18B of the ceramic ring 18 are metallized, a gold washer 20A and 20B is contacted to its metallized upper and lower faces 18A, 18B. The gold washers 20A, 20B have a thickness of about 50µ. Next, the ceramic ring 18 supporting the gold washers 20A, 20B is positioning between the first and second titanium contact rings 14 and 16. Then, the opposed titanium contact rings 14 and 16 and the intermediate ceramic ring 18 are diffusion bonded together by stacking them in a fixture capable of withstanding temperatures as high as 1,050°C. To prevent undesired bonding between the stacked titanium contact rings 14 and 16 and the ceramic ring 18 supporting the gold washers 20A, 20B and the bonding fixture, any part of the fixture in direct contact with the rings 14, 16 and 18 is composed of an electrically insulating material such as alumina, mullite, Macor, Shapal M Soft, and coated with an industrial grade lubricant, for example, a boron nitride spray.

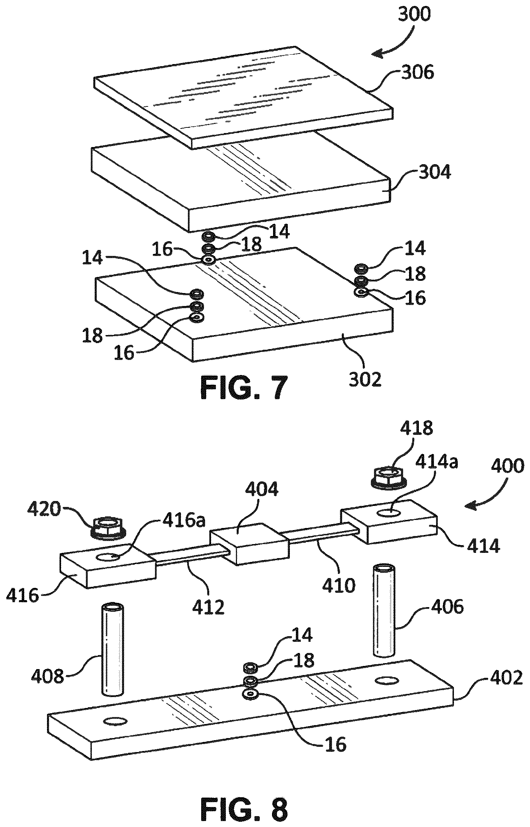

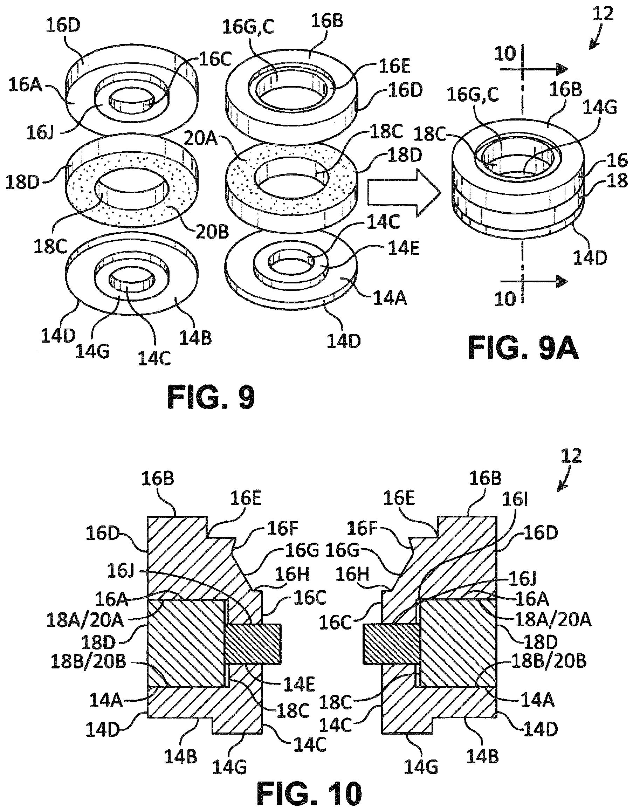

An exemplary bonding fixture 300 is illustrated in FIG. 7. The bonding fixture 300 comprises a ceramic base plate 302, a ceramic upper plate 304 and an upper dead weight 306. The base plate 302 has a lower relief depression that receives the second titanium contact ring 16 and the upper plate 304 has an upper relief depression that receives the first titanium contact ring 14. The relief depressions (not shown) in the base and upper plates 302, 304 help prevent deformation of the raised alignment features of the contact rings 14 and 16. The ceramic ring 18 supporting the gold washers 20A, 20B is positioned between the first and second titanium contact rings 14 and 16 (FIG. 9) and then the upper plate 304 is moved toward the base plate 302 until the first titanium contact ring 14/the intermediate ceramic ring 18 supporting the gold washers 20A, 20B/the second titanium contact ring 16 as a subassembly are in intimate contact with each other at their bonding interfaces. The product modular lead connector 12 is shown in FIGS. 1A and 9A.

Another exemplary bonding fixture 400 is illustrated in FIG. 8. The bonding fixture 400 comprises a ceramic base plate 402 and a ceramic upper plate 404. The base plate 402 is an elongated member having a centrally located depression (not shown) for receiving the second titanium contact ring 16. On opposite ends of the central depression, the base plate 402 supports first and second upstanding threaded bolts 406 and 408. The upper plate 404 supports opposed outwardly extending arms 410 and 412 that connect to respective upper plates 414 and 416. The upper plates 414, 416 have openings 414A and 416A that receive the threaded bolts 406, 408. The ceramic upper plate 404 also has an upper depression that receives the first titanium contact ring 14. The relief depressions (not shown) in the base and upper plates 402, 404 help prevent deformation of the raised alignment features of the titanium contact rings 14 and 16.

The ceramic ring 18 supporting the gold washers 20A, 20B is positioned between the first and second titanium contact rings 14 and 16 (FIG. 9). Nuts 418 and 420 are threaded onto the respective bolts 406, 408 and tightened to move the upper plate 404 toward the base plate 402 until the first titanium contact ring 14/the intermediate ceramic ring 18 supporting the gold washers 20A, 20B/the second titanium contact ring 16 as a subassembly are in intimate contact with each other at their bonding interfaces. The nuts 418, 420 are tightened to ensure this intimate contact. As with the bonding fixture 300 shown in FIG. 7, the magnitude of pressure and temperature needed to form a hermetic diffusion bond is at least partially dependent on the chemical composition of the materials to be bonded, the size and shape of the materials to be bonded, and the surface quality of the materials to be bonded. The resulting modular lead connector 12 is shown in FIGS. 1A and 9A.

Regardless the bonding fixture 300 (FIG. 7) or 400 (FIG. 8) that is used, a suitable hermetic diffusion bonding protocol is to expose the stacked subassembly of first titanium contact ring 14/the intermediate ceramic ring 18 supporting the gold washers 20A, 20B/the second titanium contact ring 16 to temperatures in the range of about 800°C to about 1,050°C. To prevent the buildup of undesirable stress, the temperature profile may employ a ramp rate as low as about 1°C/min to as high as about 10°C/min. Preferably, the temperature profile includes soak periods with a duration of from about 15 minutes to about 60 minutes at designated temperature intervals to reduce stress. Further, the temperature profile includes a soak period with a duration of from about 30 minutes to about 90 minutes at the highest temperature to facilitate the formation of a hermetic bond between the ceramic and titanium. A preferred heating profile gradually ramps up to a final temperature of about 950°C for a soak time of about 4 hours.

The resulting diffusion bonds at the interface of the upper titanium contact ring 14 and the ceramic ring 18 supporting the gold washers 20A and at the interface of the lower titanium contact ring 16 and the ceramic ring 18 supporting the gold washer 20B each have a thickness that is greater than 1 micron, and more preferably that is greater than about 2 microns. Undisturbed ceramic and undisturbed titanium are spaced outwardly from the greater than 1 micron diffusion bonds, and preferably the greater than about 2 microns diffusion bonds.

Field emission scanning electron microscopy (FE-SEM) confirms formation of Al₂-Au solid solution at the interface of gold and aluminum oxide with a diffusion layer thickness of about 2.5µ (the phase is classified as interpenetrating face centered cubic (FCC) CaF₂ type crystal structure) and formation of a Ti-Au intermetallic at the interface of titanium and gold, which occurs by the substitutional diffusion of titanium into the gold FCC crystal lattice with a diffusion layer thickness of about 20µ. Formation of such alloys at the micro level interface increases the bond strength and mechanical integrity of the joint compared to brazing.

FIG. 9 shows the first titanium contact ring 14/the intermediate ceramic ring 18 supporting gold washers 20A, 20B/the second titanium contact ring 16 subassembly before the various rings are contacted to each other.

FIG. 9A shows the product modular lead connector 12 which results from diffusion bonding the first titanium contact ring 14/the intermediate ceramic ring 18/the second titanium contact ring 16 subassembly shown in FIG. 9. A cross-sectional view of the diffusion bonded first titanium contact ring 14/the intermediate ceramic ring 18/the second titanium contact ring 16 subassembly is shown in FIG. 10.

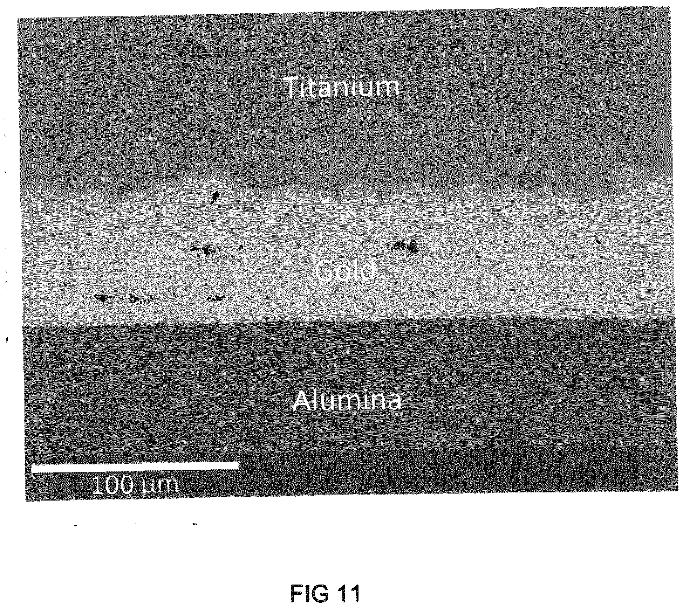

FIG. 11 is a scanning electron microscope (SEM) image showing the interface of a titanium contact ring 14, 16, an intermediary gold washer 20A, 20B, and an alumina ring 18.

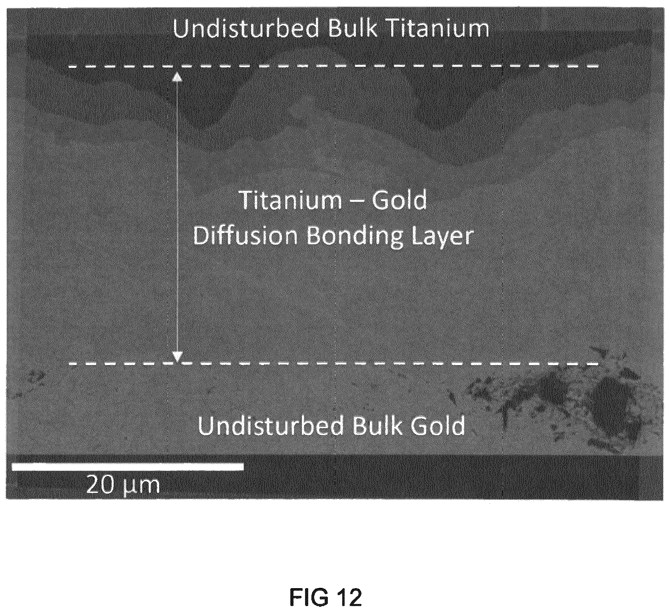

FIG. 12 is an SEM image showing the magnified interface between a titanium contact ring 14, 16 and an intermediary gold washer 20A, 20B. This SEM image also shows the presence of undisturbed titanium and undisturbed gold on opposed sides of a diffusion bond layer between the two bulk materials according to the present invention.

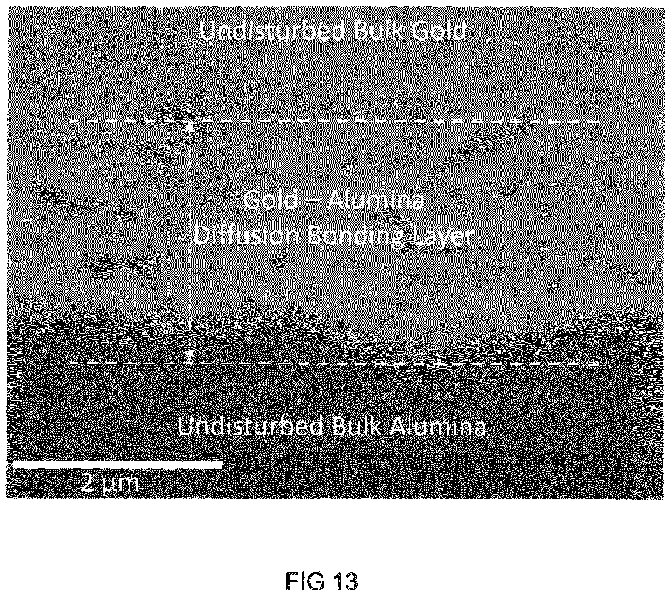

FIG. 13 is an SEM image showing the magnified interface between an alumina ring 18 and intermediary gold washer 20A, 20B. This SEM image also shows the presence of undisturbed alumina and undisturbed gold on opposed sides of a diffusion bond layer between the two bulk materials according to the present invention.

As is apparent in the photographs shown in FIGS. 11 to 13, the interfacial diffusion bond at the interface of the titanium and gold has a thickness that is greater than 15 microns, and more preferably greater than about 20 microns. The interfacial diffusion bond at the interface of the alumina and gold has a thickness that is greater than 1 micron, and more preferably greater than about 2 microns. Undisturbed ceramic and undisturbed titanium are spaced outwardly from the greater than 1 micron diffusion bond, which means that the bond at the titanium/gold/alumina interface has an interfacial toughness (strength) that is similar or equivalent to the strength of both the titanium and alumina. Moreover, the diffusion bond does not contain cracks or imperfections that are large enough to reduce the measured fracture toughness of the bond to a degree below that of the bulk fracture toughness of the alumina. Desirably, the diffusion bond is generally continuous, uniform, and crack free without any observable void formation at the interfaces, confirming a fully dense mechanically strong bond without any signs of cracking. This means that the diffusion bond is a relatively thin interface in the micro-scale range that provides a corrosion resistant and bio-stable hermetic seal between the bulk materials. The hermetic seal has a leak rate that ranges from about 0.2 to about 3.4 x 10⁻¹⁰ std cc He/s.

After the modular lead connector 12 is built, a compliant polymeric ring seal in the shape of an O-ring 22 is seated in the space between the annular protrusion 14E of the first contact ring 14 and the inner annular surface 16J of the second contact ring 16. The O-ring 22 abuts the inner annular edge 18C of the ceramic ring 18 and extends inwardly into the lumen formed by the inner annular surface 14C, 16C of the contact rings 14, 16. Silicone is a preferred material for the O-ring 22.

An annular metallic contact spring 24 is also seated against the beveled annular surface 16G of the contact ring 16. The contact spring 24 is confined in position by the opposed second and third annular steps 16F, 16H of the second titanium contact ring. A suitable contact spring 24 is a BAL SEAL^{®} type canted coil spring (BAL SEAL is a registered trademark of Bal Seal Engineering Co., Inc.).

Then, as shown in FIGS. 1 and 1A, as few as two modular lead connectors 12 to many of them, for example, 20 or more modular connectors 12 are aligned with each other and immediately adjacent connectors are welded together. FIG. 1A shows an annular weld 26 connecting the second titanium contact ring 16 of a first modular lead connector 12 to the first titanium contact ring 14 of a second modular lead connector 12.

FIG. 2 illustrates that the resulting lead connector assembly 10 is completed by welding a terminal plate 28 to the distal-most first contact ring 14. The terminal plate 28 has a centrally located circular protrusion 28A that seats against the third inner annular step 16H of the titanium contact ring 16.

Referring now back to FIG. 4B, which is an enlarged version of FIG. 4A, axis A-A is aligned along the inner annular edge 16C of the contact ring 16, axis B-B is aligned along the beveled annular surface 16G, and axis C-C is aligned along the step 16F. The angle α (alpha) between axes A-A and B-B ranges from about 25° to about 40° and is preferably about 30°, and the angle β (beta) between axes B-B and C-C ranges from about 120° to about 140° and is preferably about 130°. Further, an axis D-D aligned along the annular step 16F is at a right angle with respect to the axis A-A aligned along the inner annular edge 16C. The angular relationship between axis A-A aligned along the inner annular edge 16C, axis B-B aligned along the beveled annular surface 16G, and axis C-C aligned along the annular step 16F creates a scalene triangle (a triangle whose sides are unequal and all angles have different measures). An important aspect of the present invention is that the portion of the scalene triangle formed between axes B-B and C-C, axis D-D aligned along the annular step 16F of the second contact ring 16 and the lower protrusion 14G of the first contact ring 14 forms a pocket that confines the contact spring 24 in position as the proximal end of an implantable lead (not shown) is moved into and out of the lead connector assembly 10. For the distal-most modular lead connector 12, the contact spring 25 is confined in position by the portion of the scalene triangle formed between axes B-B and C-C, axis D-D aligned along the annular step 16F of the second contact ring 16 and the circular protrusion 28A of the terminal plate 28.

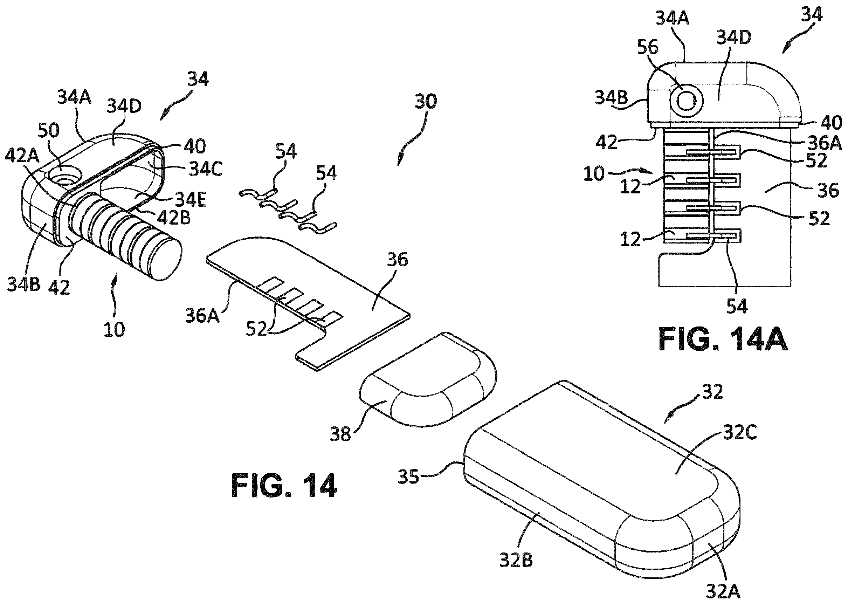

Referring now to FIG. 14, this drawing illustrates an exemplary active medical device (AMD) 30 that can be implanted in a patient's body or worn externally on a patient's body according to the present invention. The AMD 30 comprises a main housing 32 that is closed by a housing cap 34 to provide a device housing for the AMD. As will be described in greater detail hereinafter, the housing cap 34 supports the lead connector assembly 10 shown in FIGS. 1 and 1A.

The main housing 32 comprises a curved end wall 32A extending to opposed planar edge walls 32B (the opposite edge wall to wall 32B is not shown) and opposed planar face walls 32C (the opposite face wall to wall 32C is not shown). Curved intermediate sidewalls connect to the opposed housing edge walls, to the opposed housing face walls, and to the housing bottom wall. In turn, the opposed edge walls, face walls and intermediate curved sidewalls extend to an annular upper edge 35 surrounding an opening leading into an interior of the main housing 32. Titanium is a preferred material for the main housing 32.

A printed circuit board (PCB) assembly 36 comprising a PCB supporting at least two electronic circuits or electronic components (not shown) is housed inside the main housing 32. The main housing 32 also contains an electrical power source 38 connected to the PCB assembly 36 to provide electrical power to the at least two electronic circuits or electronic components. The PCB assembly 36 in turn provides electrical power to a lead (not shown) that is detachably connected to the lead connector assembly 10 shown in FIGS. 1 and 1A. As is well understood by those skilled in the art, the lead has a number of electrodes that are configured to deliver current pulses to body tissue in which the AMD 30 is implanted, receive sensed electrical signals pertaining to functions of the body tissue, or both sense electrical signals and deliver current pulses.

The electrical power source 38 for the AMD 30 can be a capacitor or a rechargeable battery, for example a hermetically sealed rechargeable Li-ion battery. However, the electrical power source 38 is not limited to any one chemistry or even a rechargeable chemistry and can be of an alkaline cell, a primary lithium cell, a rechargeable lithium-ion cell, a Ni/cadmium cell, a Ni/metal hydride cell, a supercapacitor, a thin film solid-state cell, and the like. Preferably, the electrical power source 38 is a lithium-ion electrochemical cell comprising a carbon-based or Li₄Ti₅O₁₂-based anode and a lithium metal oxide-based cathode, such as of LiCoO₂ or lithium nickel manganese cobalt oxide (LiNiₐMn_{b}Co_{1-a-b}O₂). The electrical power source 38 can also be a solid-state thin film electrochemical cell having a lithium anode, a metal-oxide based cathode and a solid electrolyte, such as an electrolyte of LiPON (LiₓPO_{y}N_{z}).

As shown in FIGS. 14, 14A, 15 and 16, the housing cap 34 comprises a curved end wall 34A extending to a planar upper edge wall 34B opposite a curved lower edge wall 34C and opposed planar face walls 34D and 34E. Curved intermediate sidewalls connect to the opposed cap upper and lower edge walls 34B, 34C, to the opposed cap left and right face walls 34D, 34E, and to the cap end wall 34A. In turn, the opposed upper and lower edge walls 34B, 34C, left and right face walls 34D, 34E and the intermediate curved sidewalls extend to an annular edge 40 surrounding an opening leading into the interior of the housing cap 34. A cap plate 42 is connected to the annular edge 40 of the housing cap 34. The cap plate 42 has a minor circular opening 42A and a major opening 42B leading into its interior.

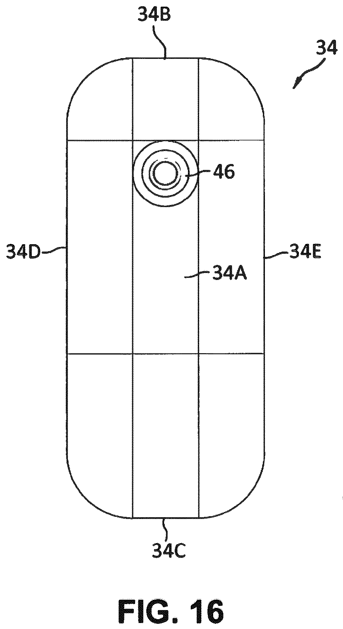

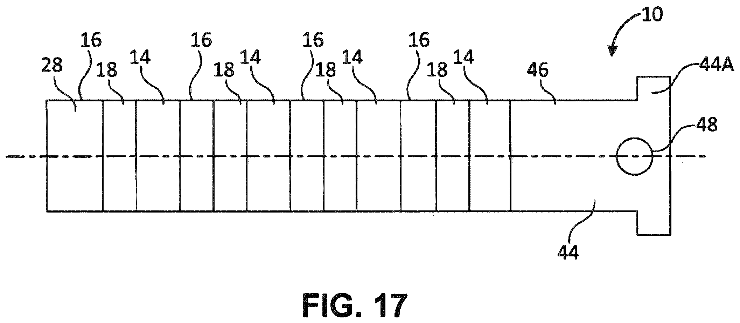

The distal end of a metallic open sleeve 44 is welded to the proximal-most first titanium contact ring 14. The open sleeve 44 also has a proximal flange 44A (FIG. 17). This flange is welded to the inner surface of the end wall 34A of the housing cap 34. This positions the main body of the lead connector assembly 10 extending outwardly beyond the annular edge 40 of the housing cap 34 and through the minor opening 42A in the cap plate 42. That way, the longitudinal axis of the lead connector assembly 10 is aligned at an orientation that is substantially perpendicular to the planar cap plate 42 and substantially perpendicular to an imaginary plane aligned along the annular edge 40 of the housing cap 34. A lead opening 46 (FIG. 16) in the curved end wall 34A of the housing cap 34 is aligned with the lumen extending through the open sleeve 44 of the lead connector assembly 10. This lumen ends at the terminal plate 28 (FIG. 2). Further, the sleeve 44 has a lateral opening 48 that aligns with a lateral treaded opening 50 in the housing cap 34. Laser or ultrasonically welding are preferred techniques for all the welded connections. Titanium is a preferred material for the sleeve 44 and terminal plate 28.

FIG. 14A shows that the PCB assembly 36 has an edge 36A that extends along an edge axis. A plurality of spaced-apart electrical contacts 52 extend to this edge. The electrical contacts are connected to the previously described at least two electronic circuits or electronic components.

To construct the AMD 30, a proximal portion of the PCB assembly 36 is moved through the major opening 42B in the cap plate 42 and into the interior of the housing cap 34. In this position, the edge axis 36A of the PCB assembly 36 is parallel to the central axis of the lumen that extends through the open sleeve 44 connected to the lead connector assembly 10. Further, the spaced-apart electrical contacts 52 of the PCB assembly 36 are aligned with the paired first and second contact rings 14, 16 connected to the metallic contact spring 24 of a modular lead connector 12 comprising the lead connector assembly 10. Jumper wires 54 connect an individual modular lead connector 12 to a corresponding electrical contact 52. The PCB assembly 36 is also connected to the electrical power source 38. In that manner, electrical continuity is established from the at least two electronic circuits or electronic components (not shown) supported on the PCB assembly 36 to the paired first and second contact rings 14, 16 connected to the metallic contact spring 24 comprising a modular lead connector 12 of the lead connector assembly 10. That is without there being a feedthrough connecting between the modular lead connector 12 and the PCB assembly 36. Feedthroughs are well known by those skilled in the art.

To close the AMD 30, the annular edge 40 of the housing cap 34 adjacent to the cap plate 42 is connected to the upper annular edge 34 of the main housing 32 to provide a hermetically closed device housing containing the electrical power source 38 connected to the PCB assembly 36.

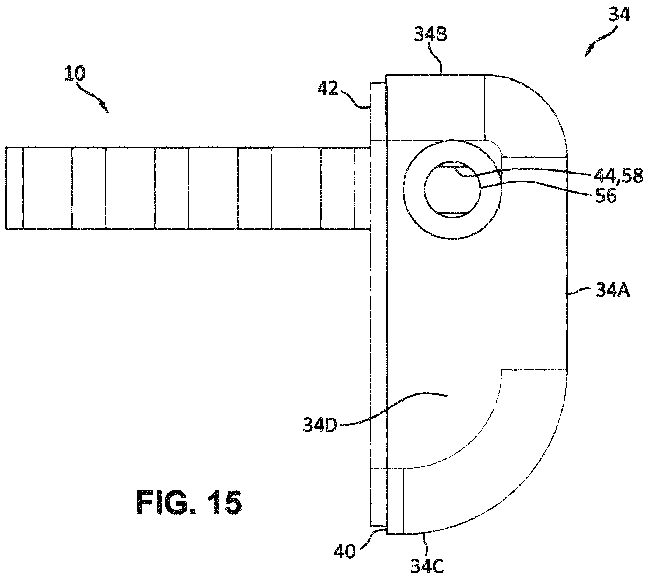

In use, the proximal end of a lead (not shown) is moved through the lead opening 46 (FIG. 16) in the curved end wall 34A of the housing cap 34, through the metallic open sleeve 44 and into the lumen provided by the lead connector assembly 10 so that electrical contacts at the proximal end of the lead are aligned with and electrically connected to the paired first and second contact rings 14, 16 connected to the metallic contact spring 24 of an individual modular lead connector 12. FIGS. 14A and 15 show that the left face wall 34D of the housing cap 34 has a lateral treaded opening 56 that is sized to receive a threaded fastener (not shown) that intersects the lateral opening 48 in the sleeve 44. That way, the fastener threaded into the aligned openings 56 and 48 contacts the proximal end of a lead to secure the lead received in the lead connector assembly 10 in place. The polymeric O-rings 22 of the lead connector assembly 10 help prevent ingress of body fluid, and the like, through the housing cap 34 to the modular lead connectors 12 of the lead connector assembly 10. In that respect, with the sleeve 44 welded to the inner surface of the housing cap 34, body fluid could flow into the lumen of the lead connector assembly 10, but the assembly 10 is impermeable to body fluid flowing through the connected modular lead connectors 12 and into the interior of the device housing formed by the housing cap 34 connected to the main housing 32.

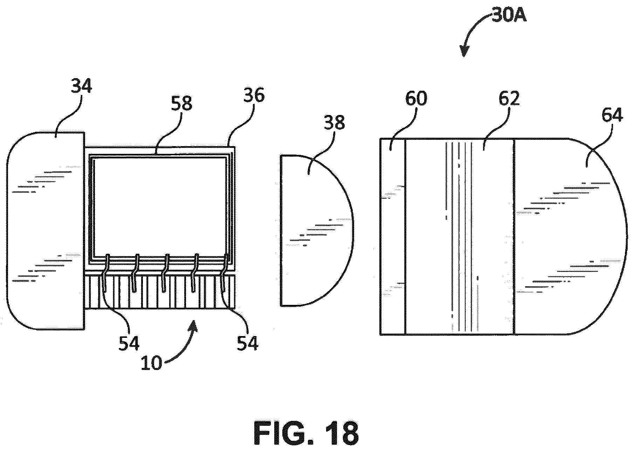

FIG. 18 is a broken-apart view of another exemplary embodiment of an AMD 30 according to the present invention. In this embodiment, the PCB assembly 36 supports a communication antenna/inductive charging coil assembly 58 that provides for remote two-way communication with an external device and inductive wireless charging of the power source 38. Whether the communication antenna/inductive charging coil assembly 58 is comprised of two dedicated antennas/coils or is an integrated antenna, it is preferably contained inside a ceramic housing for the AMD 30. The main housing 32A for the AMD 30 shown in this drawing is comprised of a proximal metallic flange 60 for connection to the housing cap 34. The flange 60 is connected to a ceramic housing portion 62 which in turn is connected to a distal metallic housing portion 64 having a closed end. Alumina is a preferred material for the ceramic housing portion 62 as it is an inherently electrically insulative material and readily permits two-way communication and inductive charging between the communication antenna/inductive charging coil assembly 58 and a remote device without creating detrimental eddy currents.

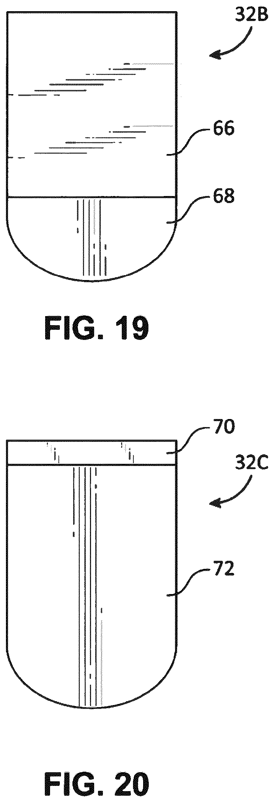

FIG. 19 illustrates another exemplary embodiment of a main housing 32B for the AMD 30. In this embodiment, the main housing 32B has a proximal portion 66 that is metallic, and which is connected to a ceramic distal housing portion 68 having a closed end. In this embodiment, the communication antenna/inductive charging coil assembly 58 is housed inside the ceramic distal housing portion 68.

FIG. 20 illustrates another exemplary embodiment of a main housing 32C for the AMD 30. The main housing 32C has a proximal metallic flange 70 for connection to the housing cap 34. The flange 70 is connected to a ceramic distal portion 72 having a closed end. In this embodiment, the communication antenna/inductive charging coil assembly 58 is housed inside the ceramic distal housing portion 72.

It is appreciated that various modifications to the inventive concepts described herein may be apparent to those skilled in the art without departing from the spirit and scope of the present invention as defined by the hereinafter appended claims.

## Claims

1. A modular lead connector for a medical device, the modular lead connector comprising:
a) first and second electrically conductive contact rings;
b) a ceramic ring having opposed first and second faces provided with respective first and second metallizations; and
c) first and second gold washers contacted to the respective first and second metallizations, wherein the ceramic ring supporting the first and second gold washers is intermediate the first and second electrically conductive contact rings, and
d) wherein the ceramic ring is in axial alignment with and hermetically connected to the respective first and second electrically conductive contact rings at respective first and second electrically conductive contact ring/ceramic ring diffusion bonds.

2. The modular lead connector of claim 1, wherein the first and second metallizations each have a thickness that ranges from about 10 nanometers to less than 1µ.

3. The modular lead connector of claim 1 or claim 2, wherein the first and second gold washers each have a thickness of about 50µ.

4. The modular lead connector of any of claims 1 to 3, wherein the ceramic ring comprises an inner annular surface spaced from an outer annular surface, the inner and outer annular surfaces being concentric and extending to opposed first and second planar faces provided with the respective first and second metallizations.

5. The modular lead connector of any of claims 1 to 4, wherein the first and second electrically conductive contact rings comprise titanium or titanium alloys and the ceramic ring comprises alumina or sapphire.

6. The modular lead connector of any of claims 1 to 5, wherein the first and second hermetic diffusion bonds of the ceramic ring supporting the first and second gold washers intermediate the respective first and second electrically conductive contact rings each have a leak rate that ranges from about 0.2 to about 3.4 x 10⁻¹⁰ std cc He/s.

7. The modular lead connector of any of claims 1 to 6, wherein the ceramic ring intermediate the first and second electrically conductive contact rings each have the same outer diameter that ranges from about 1,000µ to about 5,000µ, and wherein an inner diameter of the intermediate ceramic ring is from about 2% to about 15% larger than the inner diameters of the first and second electrically conductive contact rings which are the same and range from about 500µ to about 2,000µ,
optionally wherein an O-ring seated against an inner annular surface at the inner diameter of the ceramic ring is confined in position by the first and second electrically conductive contact rings, for instance wherein the first and second electrically conductive contact rings have respective first and second planar inner surfaces facing the intermediate ceramic ring, and wherein respective first and second raised annular alignment protrusions face each other and extend in front of the inner annular surface of the intermediate ceramic ring.

8. A lead connector assembly for a medical device, the lead connector assembly comprising:
a) at least two modular lead connectors, each modular lead connector comprising:
i) first and second titanium contact rings;
ii) an alumina ring having opposed first and second faces provided with respective first and second metallizations; and
iii) first and second gold washers contacted to the respective first and second metallizations, wherein the alumina ring supporting the first and second gold washers is intermediate the first and second titanium contact rings, and
iv) wherein the alumina ring is in axial alignment with and hermetically connected to the respective first and second titanium contact rings at respective first and second titanium/alumina diffusion bonds, and
v) wherein the at least two modular lead connectors are axially aligned and welded together; and
b) an open sleeve connected to a proximal-most one of the at least two axially aligned modular lead connectors; and
c) a terminal plate welded to a distal-most one of the at least two axially aligned modular lead connectors.

9. The lead connector assembly of claim 8, wherein the first and second metallizations provided on the opposed first and second faces of the alumina ring for the at least two axially aligned modular lead connectors each have a thickness that ranges from about 10 nanometers to less than 1µ.

10. The lead connector assembly of claim 8 or claim 9, wherein the first and second gold washers contacted to the respective first and second metallizations provided on the opposed first and second faces of the alumina ring for each of the at least two axially aligned modular lead connectors have a thickness of about 50µ.

11. The lead connector assembly of any of claims 8 to 10, wherein the alumina ring for each of the at least two axially aligned modular lead connectors comprises an inner annular surface spaced from an outer annular surface, the inner and outer annular surfaces being concentric and extending to opposed first and second planar faces provided with the respective first and second metallizations.

12. The lead connector assembly of any of claims 8 to 11, wherein the first and second hermetic diffusion bonds of the alumina ring supporting the first and second gold washers intermediate the respective first and second titanium contact rings for the at least two axially aligned modular lead connectors each have a leak rate that ranges from about 0.2 to about 3.4 x 10⁻¹⁰ std cc He/s.

13. The lead connector assembly of any of claims 8 to 12, wherein the first and second titanium contact rings and the intermediate alumina ring each have the same outer diameter that ranges from about 1,000µ to about 5,000µ, and wherein an inner diameter of the intermediate alumina ring is from about 2% to about 15% larger than the inner diameters of the first and second titanium contact rings which are the same and range from about 500µ to about 2,000µ,
optionally wherein an O-ring seated against an inner annular surface at the inner diameter of the alumina ring is confined in position by the first and second titanium contact rings, for instance wherein the first and second titanium contact rings have respective first and second planar inner surfaces facing the intermediate alumina ring, and wherein respective first and second raised inner annular alignment protrusions face each other and extend radially inwardly in front of the inner annular surface of the intermediate alumina ring.

14. The lead connector assembly of any of claims 8 to 13, wherein the at least two axially aligned modular lead connectors welded together comprise a first modular lead connector and an adjacent second modular lead connector, and wherein the first titanium contact ring of the first modular lead connector has a first raised outer annular alignment protrusion that is received in a second outer annular step of the second titanium contact ring of the adjacent second modular lead connector, and wherein the second titanium contact ring of the second modular lead connector further comprises a second intermediate inner annular step that is spaced radially inwardly from the second outer annular step and which meets a second beveled annular surface which in turn meets a second inner annular step that is spaced radially inwardly from the second beveled annular surface, and wherein an annular metallic contact spring is seated against the second beveled annular surface extending to the opposed second intermediate inner annular step and the second inner annular step of the second modular lead connector and the first raised outer annular alignment protrusion of the first titanium contact ring of the fist modular lead connector.

15. An active medical device (AMD), comprising:
a) a device housing comprising a housing sidewall defining an open housing interior, the housing sidewall extending to a device housing annular rim;
b) a printed circuit board (PCB) assembly comprising a printed circuit board supporting at least one electronic component, wherein the PCB assembly is housed inside the housing interior;
c) an electrical power source housed inside the housing interior and connected to the PCB assembly;
d) a housing cap comprising a housing cap sidewall defining an open housing cap interior, the housing cap sidewall extending to an housing cap annular rim, wherein the housing cap sidewall comprising a lead opening; and
e) a lead connector assembly, comprising:
i) at least two modular lead connectors, each modular lead connector comprising:
A) first and second titanium contact rings;
B) an alumina ring having opposed first and second faces provided with respective first and second metallizations; and
C) first and second gold washers contacted to the respective first and second metallizations, wherein the alumina ring supporting the first and second gold washers is intermediate the first and second titanium contact rings, and
D) wherein the alumina ring is in axial alignment with and hermetically connected to the respective first and second titanium contact rings at respective first and second titanium/alumina diffusion bonds; and
ii) wherein the at least two modular lead connectors are axially aligned and welded together; and
iii) an open sleeve connected to a proximal-most one of the at least two axially aligned modular lead connectors; and
iv) a terminal plate welded to a distal-most one of the at least two axially aligned modular lead connectors,
v) wherein the open sleeve of the lead connector assembly is welded to an interior surface of the housing cap aligned with the lead opening; and
f) at least one jumper wire extending from at least one of a second titanium contact ring welded to a first titanium contact ring of the respective at least two modular lead connectors to the PCB assembly housed inside the housing interior and connected to the electrical power source.

16. The AMD of claim 15, further comprising a communication antenna/inductive charging coil assembly electrically connected to the at least one electronic component of the PCB assembly.
